# FASCICULE DE BREVET EUROPEEN

(11) **EP 1 003 907 B1**
(45) Date de publication et mention de la délivrance du brevet: **14.11.2007**
(21) Numéro de dépôt: 98941461.0
(22) Date de dépôt: 27.07.1998
(51) Int. Cl.: C12Q 1/48

(54) **METHODES ET KITS POUR LE DIAGNOSTIC DE LA MALADIE D'ALZHEIMER**
VERFAHREN UND TESTSÄTZE ZUR DIAGNOSE VON MORBUS ALZHEIMER
METHODS AND KITS FOR DIAGNOSING ALZHEIMER DISEASE

(30) Priorité: 31.07.1997 FR 9709823
(43) Date de publication de la demande: 31.05.2000
(73) Titulaire: CENTRE NATIONAL DE LA RECHERCHE SCIENTIFIQUE, 75016 Paris Cédex (FR)
(72) Inventeur: JANOSHAZI, Agnès, F-67000 Strasbourg (FR); DE BARRY, Jean, F-67100 Strasbourg (FR)
(74) Mandataire: Peaucelle, Chantal
(86) Numéro de dépôt international: PCT/FR1998/001660
(87) Numéro de publication internationale: WO 1999/006590

(56) Documents cités:
- WO-A-93/04194
- WO-A-97/07402
- WO-A-97/11094
- US-A- 4 874 694
- MASLIAH E ET AL: "PROTEIN KINASE C ALTERATION IS AN EARLY BIOCHEMICAL MARKER IN ALZHEIMER'S DISEASE" JOURNAL OF NEUROSCIENCE, vol. 11, no. 9, septembre 1991, pages 2759-2767, XP002046527
- KRAFFT G. A. & WANG G. T.: "Fluorescent probes in studies of proteases." ACS SYPOSIUM SERIES - FLUORESCENT CHEMOSENSORS FOR ION AND MOLECULE RECOGNITION., vol. 538, 1993, pages 183-195, XP002062970
- CHII-SHIARNG CHEN AND MARTIN POENIE: "New Fluorescent Probes for Protein Kinase C. Synthesis, Characterization and Application." THE JOURNAL OF BIOLOGICAL CJEMISTRY, vol. 268, no. 21, 1993, pages 15812-15822,

## Description

L'invention a pour objet une méthode et des kits pour le diagnostic de la maladie d'Alzheimer.

L'accroissement général de la longévité favorise la fréquence de la maladie d'Alzheimer et cette pathologie constitue pour les sociétés modernes un problème économique majeur. Un effort de recherche important est réalisé pour comprendre la génèse de cette maladie, mais les tentatives pour trouver un marqueur précoce ont été infructueuses ou incomplètes. L'examen clinique est le plus souvent utilisé seul, et le diagnostic sûr n'est posé qu'après biopsie cérébrale ou autopsie.

En 1991 Masliah et coll (J. Neuroscience, 11, 2759-2767) ont publié des éléments suggèrant que des anomalies de l'activité enzymatique de la PKC étaient constatées exclusivement dans le système nerveux central, associées aux stades initiaux de la maladie d'Alzheimer. Ces travaux ne faisaient pas état d'anomalies de la PKC constatées dans les cellules sanguines.

En 1989, Gandy et Greengard (US4874694) ont proposé de détecter les maladies neurodégénératives, et en particulier la maladie d'Alzheimer, en analysant, par marquage radioactif et électrophorèse, les protéines présentes dans des échantillons de liquide céphalorachidien. Cette technique, invasive est difficile à mettre en oeuvre. Elle utilise la PKC comme réactif. Elle ne démontre pas une anomalie de la PKC associée à la maladie d'Alzheimer. En outre elle utilise un échantillon provenant du système nerveux central et n'apporte aucun élément concernant les organes périphériques, et en particulier les éléments présents dans le sang.

Récemment, une équipe américaine a proposé l'utilisation d'antagonistes cholinergiques dans la mesure de la dilatation de la pupille comme test de dépistage (Scinto et al., 1994, Science 266:1051-1054). Ce test cependant ne permet pas une discrimination absolue des patients atteints par la maladie d'Alzheimer. Son intérêt diagnostique a été fortement remis en cause dans plusieurs études plus récentes.

Enfin, il a également été montré récemment que le sérum provenant de patients atteints par la maladie d'Alzheimer contenait des anticorps susceptibles de reconnaître des antigènes exprimés par des cellules microgliales de souris. Les raisons de cette propriété et sa valeur diagnostique ne sont pas pour l'instant établies.

L'observation de diverses anomalies chez les patients atteints de la maladie d'Alzheimer a amené les inventeurs à étudier les relations entre ces anomalies et les modifications des niveaux d'activation de certaines enzymes.

Ainsi, prenant en compte les relations précédemment établies entre une anomalie du transport de chlorure dans les érythrocytes, chez les patients atteints de la maladie d'Alzheimer, et une anomalie de l'activité de la protéine kinase C (PKC en abrégé), leurs travaux ont porté dans un premier temps sur la mesure du niveau d'activation de la PKC sur des érythrocytes de tels patients.

Les inventeurs ont alors constaté qu'en faisant varier les conditions de mesure, il était possible de disposer de données permettant de discriminer les patients atteints de la maladie des sujets sains.

Le développement de leurs travaux a permis de vérifier que de telles mesures appliquées à la PKA, ou encore mettant en oeuvre un jeu des composés susceptibles d'interagir avec la PKC ou la PKA, étaient également exploitables pour discriminer les patients d'Alzheimer.

L'invention a donc pour but de fournir une méthode de diagnostic de la maladie d'Alzheimer permettant d'établir un test fiable, rapidement, et à moindre coût.

Elle vise également à fournir des kits permettant une mise en oeuvre aisée d'une telle méthode.

La méthode de diagnostic de la maladie d'Alzheimer selon l'invention est caractérisée en ce qu'elle comprend :
- l'incubation d'un échantillon sanguin avec une sonde fluorescente reconnaissant le site catalytique de la protéine kinase C (PKC) ou de la protéine kinase A (PKA) ; ou avec deux sondes fluorescentes reconnaissant respectivement le site catalytique et le domaine régulateur d'une desdites enzymes (PKC ou PKA), dans des conditions permettant la liaison spécifique de la ou des sondes avec PKC ou PKA,
   ladite sonde reconnaissant le domaine régulateur de PKC ou PKA comprenant des composés pouvant assurer une émission dans le domaine d'excitation de la sonde interagissant avec le domaine catalytique de ladite enzyme,
- la mesure de l'intensité de fluorescence de la préparation obtenue et de ses variations éventuelles par addition d'inhibiteur et/ou d'activateur de PKC ou PKA, une variation de données des spectres d'émission de fluorescence, par rapport à celles de spectres obtenus avec des sujets sains, étant discriminante vis-à-vis de la maladie d'Alzhzeimer,
   ladite mesure de l'intensité de fluorescence de la préparation obtenue se faisant, dans le cas de l'incubation d'un échantillon sanguin avec les deux sondes fluorescentes reconnaissant respectivement le site catalytique et le domaine régulateur de PKC ou PKA, par excitation dudit échantillon à la longueur d'onde d'absorption de la sonde fluorescente reconnaissant le site catalytique de ladite enzyme, puis observation du signal obtenu à la longueur d'onde d'émission de ladite sonde catalytique, qui est discriminant vis-à-vis de la maladie d'Alzhzeimer.

Conformément à l'invention, pour étudier les intensités de fluorescence on effectue une déconvolution des spectres d'émission obtenus, pour les décomposer en gaussiennes élémentaires et déterminer les pics d'émission caractéristiques de l'interaction, directe ou indirecte, de la sonde avec la PKC ou la PKA. La détermination de la contribution relative de chaque gaussienne à l'intensité globale de fluorescence dans des conditions différentes, permet de sélectionner les critères à retenir pour effectuer le diagnostic. Il s'agit par exemple d'une différence d'intensité relative ou d'un glissement d'un pic par rapport aux résultats obtenus avec des sujets sains.

On dispose ainsi de moyens permettant d'établir une discrimination de 100 % entre les patients atteints de la maladie d'Alzheimer et des sujets sains.

Les sondes fluorescentes utilisables dans la méthode de l'invention comportent, couplé à un fluorophore, un composé capable d'interagir, dans les conditions d'incubation, avec le site catalytique de l'enzyme, ou son domaine régulateur.

Des sondes fluorescentes capables de réagir, dans les conditions de l'invention, avec le site catalytique de la PKC, comprennent des composés organiques de synthèse, tels que des dérivés de bis-indolylmaléimide. On citera en particulier les sondes fim-1 et rim-1 décrites par Chen et Poenie dans J. Biol. Chem, 1993, 268, 15812-15822.

D'autres sondes fluorescentes permettent le marquage de composés capables d'interagir, dans les conditions de l'invention, avec le domaine régulateur de la PKC.

Il s'agit en particulier de sondes lipidiques, telles qu'élaborées à partir de phospholipides constituants de la membrane, ou dérivés de tels phospholipides.

En variante, les sondes fluorescentes sont des sondes protéiques ou peptidiques de nature membranaire ou cytosquelettique, ou dérivées des substrats des enzymes PKC ou PKA.

De telles sondes lipidiques, protéiques ou peptidiques sont utilisées simultanément avec les sondes reconnaissant le domaine catalytique des enzymes PKC. On assure ainsi la spécificité des mesures, des enzymes ou protéines autres que PKC ou PKA étant susceptibles d'interagir avec les composés lipidiques, protéiques ou peptidiques des sondes.

Ces sondes comprennent par exemple des composés assurant une émission dans le domaine d'excitation des sondes interagissant avec le domaine catalytique des enzymes.

Leur excitation entraîne à son tour celle de la sonde reconnaissant le site catalytique, ce qui implique une proximité des deux sondes (moins de 50 environ), l'enzyme interagissant alors directement avec les éléments de la membrane ou du cytosquelette ou du substrat.

Ainsi, en excitant l'échantillon à étudier à la longueur d'onde d'absorption de la sonde lipidique, et en observant le signal à la longueur d'onde d'émission de la sonde catalytique, on dispose d'un signal spécifique vis-à-vis de l'enzyme et permettant d'établir la discrimination.

Par "échantillon sanguin", on entend l'échantillon total prélevé sur le patient, ou une fraction de cet échantillon, ou des éléments constitutifs, par exemple les érythrocytes.

De manière générale, pour la mise en oeuvre de la méthode de l'invention, on utilise un échantillon de sang frais.

L'étape d'incubation est réalisée à température ambiante, en chargeant l'échantillon à étudier avec une quantité de sonde, et selon une durée, permettant d'obtenir l'interaction souhaitée.

Dans un mode préféré de réalisation de l'invention, une partie de l'échantillon est mise à incuber avec la sonde seule, une autre partie est tout d'abord mise à incuber avec un inhibiteur de la PKC ou de la PKA, puis avec la sonde, et une autre partie est préalablement incubée avec un activateur de la pKC ou de la pKA, puis avec la sonde.

A titre d'exemple, on citera, comme inhibiteur de la PKC, la staurosporine, et comme activateur, un ester de phorbol, comme l'acétate-myristate de phorbol (PMA ou TPA)

La déconvolution des spectres obtenus permet d'identifier des pics caractéristiques des interactions. La comparaison des spectres en présence ou non d'activateur et ou d'inhibiteur de l'enzyme permet de relever des variations chez les patients atteints de la maladie d'Alzheimer par rapport à des sujets sains, et d'établir rapidement une discrimination nette entre ces groupes.

L'invention vise également des kits ou trousses de diagnostic pour la mise en oeuvre du test défini ci-dessus.

Ces kits ou trousses pour le diagnostic de la maladie d'Alzheimer sont caractérisés en ce qu'ils comportent :
- une notice d'utilisation,
- deux sondes fluorescentes reconnaissant, respectivement, le site catalytique et le domaine régulateur d'une desdites enzymes PKC ou PKA, avec le site catalytique de l'enzyme, ou avec son domaine régulateur.

Ces kits sont caractérisés en ce que les sondes fluorescentes comportant un composé capable d'interagir avec le domaine régulateur de la PKC sont telles qu'élaborées à partir de phospholipides, les sondes protéiques ou peptidiques de nature membranaire ou cytosquelettique, ou dérivées des substrats des enzymes PKC ou PKA.

L'invention fournit ainsi des moyens de grande fiabilité non invasifs, permettant de réaliser rapidement et économiquement un diagnostic de la maladie d'Alzheimer.

En développant des outils informatiques pour l'analyse des spectres enregistrés, il est possible d'effectuer très rapidement des mesures sur des groupes importants de sujets susceptibles de développer la maladie et sur des familles dont certains membres présentent des formes héréditaires.

D'autres caractéristiques et avantages de l'invention sont rapportés dans les exemples qui suivent et en se référant aux figures 1 à 6.

Dans ces figures,
- la figure 1 représente la déconvolution du spectre d'émission de fim-1,
- la figure 2, les différences d'intensité relative des pics d'émission de fluorescence à 518, 535, 547 et 585 nm de préparations d'érythrocytes pré-incubées avec de la staurosporine, puis additionnées de fim-1,
- la figure 3, le glissement du deuxième pic en comparant les échantillons de patients Alzheimer et de volontaires sains,
- la figure 4, les différences d'intensité relative des pics d'émission de fluorescence à 518, 535, 545 et 585 nm de préparations d'érythrocytes pré-incubées avec fim-1, puis additionnées de TPA,
- les figures 5 et 6, les différences d'intensité relative des pics d'émission de fluorescence de préparations d'érythrocytes de patients sains et de patients atteints de la maladie de Parkinson, pré-incubées avec de la staurosporine puis additionnées de fim-1 (figure 5), ou pré-incubées avec fim-1, puis additionnées de TPA (figure 6).

### Exemple 1 : Etude des propriétés de fluorescence de fim-1

On utilise des liposomes préparés à partir d'extraits lipidiques de cerveau et une préparation commerciale de PKC α purifiée, puis on ajoute des érythrocytes.

Cette préparation est incubée 20 min à température ambiante, en présence de fim-1 (500 nM) dans un tampon PBS (150 mM NaCl, 10 mM NaHPO₄, 5 mM D-glucose, 500- µM CaCl₂, pH 7,4).

Après lavage, centrifugation et remise en suspension dans PBS 1/150, on enregistre le spectre d'émission de fluorescence entre 505 et 650 nm, avec une longueur d'onde d'excitation de 485 nm.

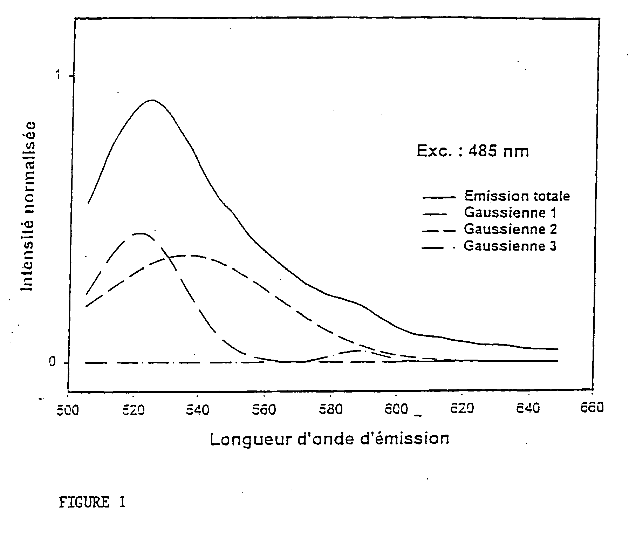

Le spectre obtenu est normalisé par rapport à l'intensité de fluorescence mesurée avec la sonde seule, puis décomposé en 3 gaussiennes élémentaires représentées sur la figure 1. On note un maximum d'émission respectivement à 518, 535 et 586 min. Les interactions entre fim-1 et les lipides membranaires ainsi que la PKC se traduisent par un léger glissement du spectre entre 530 et 570 nm pour les lipides, et une augmentation de la fluorescence entre 510 et 530 nm, et au-delà de 570 nm, pour la PKC.

L'addition de staurosporine inhibiteur compétitif du site ATP, se traduit par une inhibition de l'intensité de fluorescence. En revanche, on note une augmentation considérable de la fluorescence (le rendement quantique augmente d'un facteur de 5000) en présence d'ester de phorbol.

### Exemple 2 : test de diagnostic de la maladie d'Alzheimer.

### . protocole

1 à 3 ml de sang frais sont prélevés sur héparine et conservés au froid (+4°C). Dans les deux heures qui suivent l'échantillon est lavé et centrifugé trois fois dans du PBS à température ambiante.

On procède alors à 3 séries d'incubation :
- un premier aliquote de cellules incubé 20 min en présence de la sonde fluorescente (500 nM) fim-1,
- un deuxième aliquote d'abord pré-incubé en présence de 500 nM staurosporine pendant 20 min, puis en présence de la sonde fluorescente, et
- un troisième aliquote d'abord pré-incubé en présence de la sonde fluorescente, puis de 500 nM de TPA pendant 20 mn.

A la suite de ces incubations les cellules sont lavées, centrifugées (deux fois), puis remises en suspension dans du PBS (1/150).

Les spectres d'émission de fluorescence respectifs sont mesurés sur un volume d'environ 200 µl avec un spectrofluorimètre SLM 4800 à faisceau unique (Ex 485 nm, largeur de fente 2 nm), à température ambiante. Chaque mesure est effectuée trois fois au moins en double, puis une moyenne des valeurs est établie.

Les spectres sont ensuite normalisés par rapport à l'intensité de fluorescence mesurée avec la sonde seule, puis décomposés en trois gaussiennes élémentaires. L'intégrale de chaque gaussienne est enfin mesurée et rapportée à la somme des intégrales des gaussiennes pour chaque spectre (contribution relative de chaque gaussienne à l'intensité globale de fluorescence).

### . discrimination entre des patients Alzheimer et des sujets sains

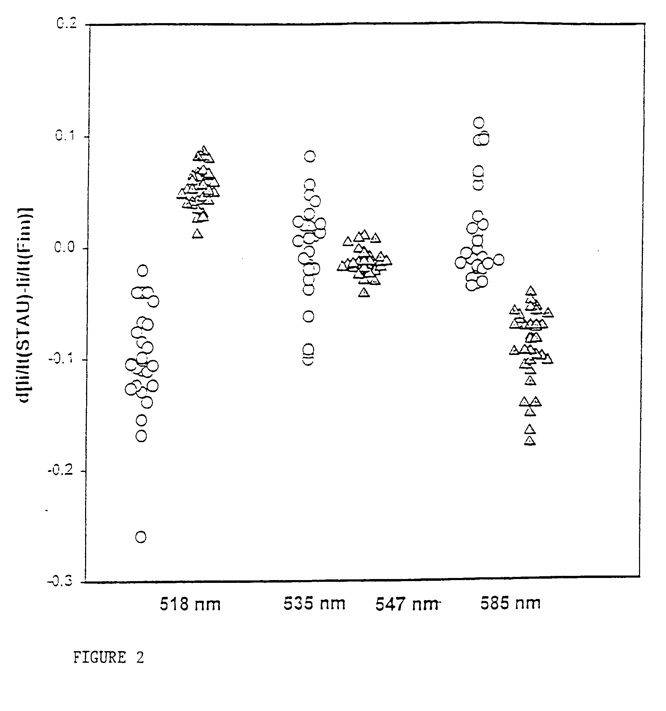

La mesure des spectres d'émission de fluorescence de fim-1 dans des globules rouges provenant de 35 patients atteints de la maladie d'Alzheimer et leur comparaison avec celles obtenues à partir de globules rouges provenant de 35 volontaires sains d'âge comparable a permis une discrimination de 100 % entre les 2 groupes sur deux critères : la différence d'intensité du pic de fluorescence à 518 nm lorsque le spectre est mesuré en présence ou en l'absence de staurosporine (figure 2), et le glissement du maximum du pic à 545 nm.

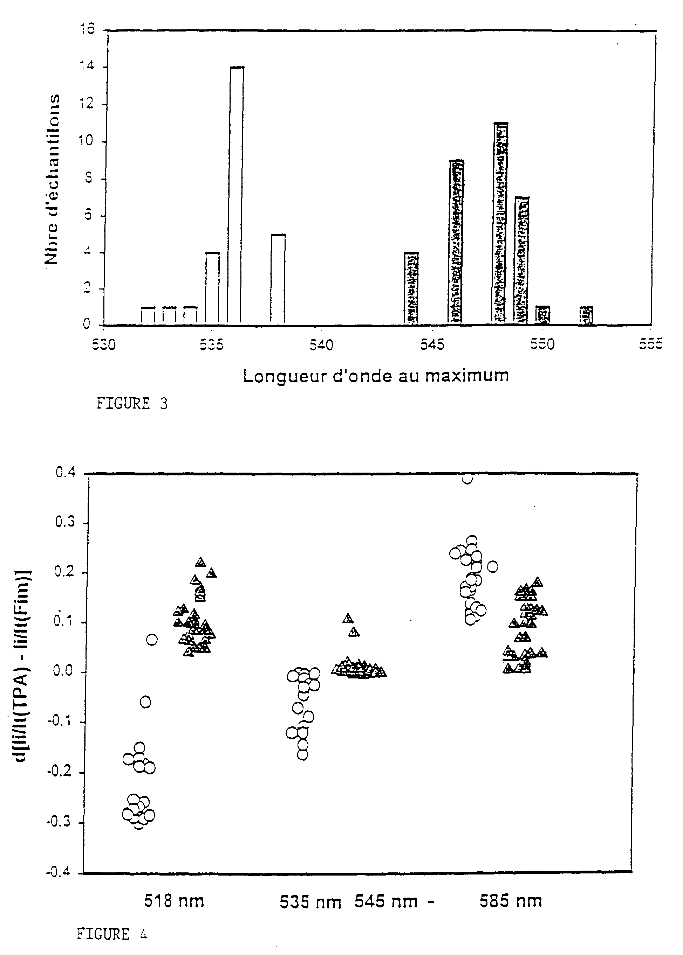

Sur ces figures ▲ et ■ correspondent aux patients d'Alzheimer et O et □ aux sujets sains. Ces symboles ont les mêmes significations dans la figure 4 qui représente les résultats obtenus en présence ou en l'absence d'ester de phorbol.

### . mesures effectuées sur des patients atteints de la maladie de Parkinson

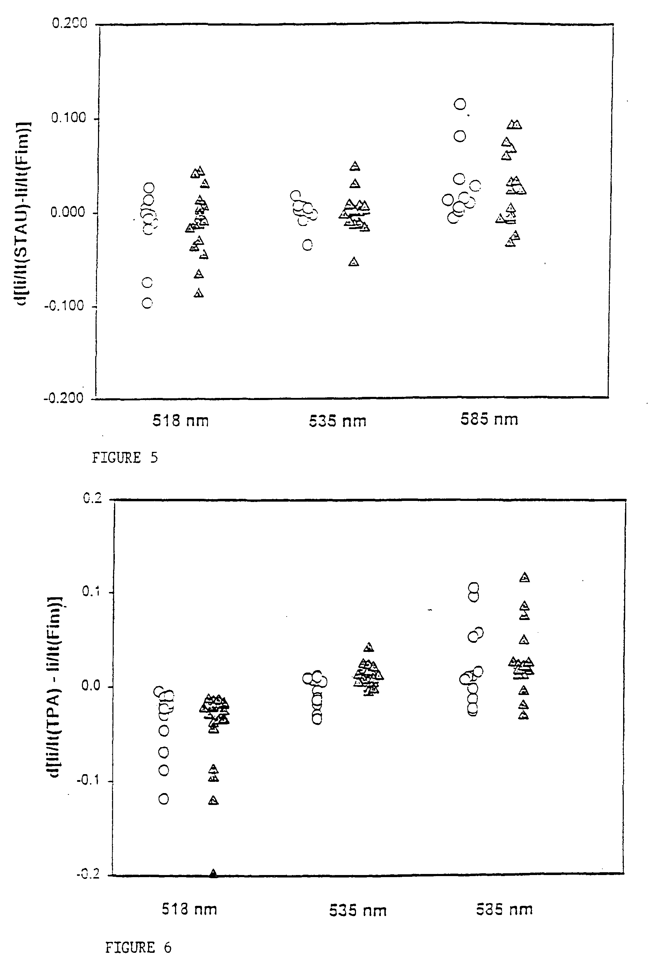

De façon à établir la spécificité des mesures pour la maladie d'Alzheimer par rapport à d'autres maladies neurodégénératives, on a procédé à des mesures sur des échantillons provenant de 15 patients atteints de la maladie de Parkinson ou de volontaires sains d'âge comparable. Les deux index de mesure retenus n'ont pas permis d'observer de différence entre les volontaires sains et les patients (effet de la staurosporine : figure 5 et effet du TPA : figure 6 où "O" correspond aux volontaires sains et "▲" aux patients Parkinson). Ce résultat est un bon indice de la spécificité du test de l'invention pour un diagnostic de la maladie d'Alzheimer.

## Revendications

1. Méthode de diagnostic de la maladie d'Alzheimer, **caractérisée en ce qu'**elle comprend :
- l'incubation d'un échantillon sanguin avec une sonde fluorescente reconnaissant le site catalytique de la protéine kinase C (PKC) ou de la protéine kinase A (PKA) ; ou avec deux sondes fluorescentes reconnaissant respectivement le site catalytique et le domaine régulateur d'une desdites enzymes (PKC ou PKA), dans des conditions permettant la liaison spécifique de la ou des sondes avec PKC ou PKA,
ladite sonde reconnaissant le domaine régulateur de PKC ou PKA comprenant des composés pouvant assurer une émission dans le domaine d'excitation de la sonde interagissant avec le domaine catalytique de ladite enzyme,
- la mesure de l'intensité de fluorescence de la préparation obtenue et de ses variations éventuelles par addition d'inhibiteur et/ou d'activateur de PKC ou PKA, une variation de données des spectres d'émission de fluorescence, par rapport à celles de spectres obtenus avec des sujets sains, étant discriminante vis-à-vis de la maladie d'Alzhzeimer,
ladite mesure de l'intensité de fluorescence de la préparation obtenue se faisant, dans le cas de l'incubation d'un échantillon sanguin avec les deux sondes fluorescentes reconnaissant respectivement le site catalytique et le domaine régulateur de PKC ou PKA, par excitation dudit échantillon à la longueur d'onde d'absorption de la sonde fluorescente reconnaissant le site catalytique de ladite enzyme, puis observation du signal obtenu à la longueur d'onde d'émission de ladite sonde catalytique, qui est discriminant vis-à-vis de la maladie d'Alzhzeimer.

2. Méthode selon la revendication 1, **caractérisée en ce que** les sondes fluorescentes reconnaissant le site catalytique de la PKC ou de la PKA et celles reconnaissant son domaine régulateur, comportent, couplé à un fluorophore, un composé capable d'interagir, dans les conditions d'incubation, respectivement, avec le site catalytique de l'enzyme, ou avec son domaine régulateur.

3. Méthode selon la revendication 2, **caractérisée en ce que** les sondes fluorescentes capables de réagir, dans les conditions de l'invention, avec le site catalytique de la PKC, choisis parmi des composés organiques de synthèse, tels que des dérivés de bis-indolylmaléimide.

4. Méthode selon la revendication 2, **caractérisée en ce qu'**on utilise les sondes fluorescentes comportant un composé capable d'interagir, dans les conditions de l'invention, avec le domaine régulateur de la PKC, sont telles qu'élaborées à partir de phospholipides constituants de la membrane ou dérivés de tels phospholipides, les sondes protéiques ou peptidiques de nature membranaire ou cytosquelettique, ou dérivées des substrats des enzymes PKC ou PKA.

5. Méthode selon la revendication 4, **caractérisée en ce qu'**on utilise simultanément les sondes reconnaissant le domaine régulateur de PKC ou de PKA et les sondes reconnaissant directement le domaine catalytique des enzymes PKC.

6. Méthode selon l'une quelconque des revendications 1 à 5, **caractérisée en ce que** l'étape d'incubation est réalisée à température ambiante.

7. Méthode selon l'une quelconque des revendications précédentes, **caractérisée en ce qu'**une partie de l'échantillon est mise à incuber avec la ou les sondes seules, une autre partie est tout d'abord mise à incuber avec un inhibiteur de la PKC ou de la PKA, puis avec la ou les sondes, et une autre partie est préalablement incubée avec la ou les sondes, puis avec un activateur de la PKC ou de la PKA.

8. Kits ou trousses pour le diagnostic de la maladie d'Alzheimer, **caractérisés en ce qu'**ils comportent:
- une notice d'utilisation,
- deux sondes fluorescentes reconnaissant, respectivement, le site catalytique et le domaine régulateur d'une desdites enzymes PKC ou PKA, et
- des réactifs pour la réalisation du test de diagnostic, ces réactifs étant choisis parmi des activateurs et/ou des inhibiteurs de la PKC et/ou de la PKA,
ou, à la place des deux sondes fluorescentes, une seule sonde fluorescente telle que définie ci-dessus avec, en tant que réactifs c), des inhibiteurs de la PKC et/ou de la PKA.

9. Kits ou trousses selon la revendication 8, **caractérisés en ce que** les sondes reconnaissant respectivement le site catalytique et le domaine régulateur d'une desdites enzymes PKC ou PKA comportent, couplé à un fluorophore, un composé capable d'interagir, dans les conditions d'incubation, respectivement, avec le site catalytique de l'enzyme, ou avec son domaine régulateur.

10. Kits selon la revendication 9, **caractérisés en ce que** les sondes fluorescentes comportant un composé capable d'interagir avec le domaine régulateur de la PKC sont des phospholipides constituants de la membrane, des sondes protéiques ou peptidiques de nature membranaire ou cytosquelettique, ou des dérivées des substrats des enzymes PKC ou PKA.

## Claims

1. Method for diagnosing Alzheimer's disease, **characterized in that** it comprises:
- the incubation of a blood sample with a fluorescent probe recognizing the catalytic site of protein kinase C (PKC) or protein kinase A (PKA); or with two fluorescent probes recognizing respectively the catalytic site and the regulatory domain of one of said enzymes (PKC or PKA), under conditions permitting the specific binding of the probe or probes with PKC or PKA,
said probe recognizing the regulatory domain of PKC or PKA comprising compounds that can ensure an emission in the excitation domain of the probe interacting with the catalytic domain of said enzyme,
- the measurement of the intensity of fluorescence of the preparation obtained and of its possible variations by the addition of an inhibitor and/or activator of PKC or PKA, a variation of fluorescence emission spectra data, compared with those of spectra obtained with healthy subjects, being discriminating vis-à-vis Alzheimer's disease,
said measurement of the intensity of fluorescence of the preparation obtained taking place, in the case of the incubation of a blood sample with the two fluorescent probes recognizing respectively the catalytic site and the regulatory domain of PKC or PKA, by excitation of said sample at the absorption wavelength of the fluorescent probe recognizing the catalytic site of said enzyme, then observation of the signal obtained at the emission wavelength of said catalytic probe, which is discriminating vis-a-vis Alzheimer's disease.

2. Method according to claim 1, **characterized in that** the fluorescent probes recognizing the catalytic site of PKC or PKA and those recognizing its regulatory domain comprise, coupled with a fluorophore, a compound capable of interacting, under the incubation conditions, respectively with the catalytic site of the enzyme or with its regulatory domain.

3. Method according to claim 2, **characterized in that** the fluorescent probes capable of reacting, under the conditions of the invention, with the catalytic site of PKC, are chosen from organic synthesis compounds, such as derivatives of bis-indolylmaleimide.

4. Method according to claim 2, **characterized in that** fluorescent probes comprising a compound capable of interacting, under the conditions of the invention, with the regulatory domain of the PKC are used as developed from phospholipids constituting the membrane or derived from such phospholipids, proteic or peptidic probes of membranous or cytoskeletal nature, or derived from the substrates of the PKC or PKA enzymes.

5. Method according to claim 4, **characterized in that** the probes recognizing the regulatory domain of PKC or PKA and the probes directly recognizing the catalytic domain of the PKC enzymes are used simultaneously.

6. Method according to any one of claims 1 to 5, **characterized in that** the incubation stage is carried out at ambient temperature.

7. Method according to any one of the previous claims, **characterized in that** part of the sample is incubated with the probe or probes alone, another part is first of all incubated with an inhibitor of PKC or PKA, then with the probe or probes, and another part is incubated beforehand with the probe or probes, then with an activator of PKC or PKA.

8. Kits or sets for diagnosing Alzheimer's disease, **characterized in that** they comprise:
- instructions for use,
- two fluorescent probes recognizing, respectively, the catalytic site and the regulatory domain of one of said enzymes PKC or PKA,
and
- reagents for carrying out the diagnostic test, these reagents being chosen from the activators and/or inhibitors of PKC and/or PKA,
or, instead of the two fluorescent probes, a single fluorescent probe as defined above with, as reagents c), inhibitors of PKC and/or PKA.

9. Kits or sets according to claim 8, **characterized in that** the probes recognizing respectively the catalytic site and the regulatory domain of one of said enzymes PKC or PKA comprise, coupled to a fluorophore, a compound capable of interacting, under the incubation conditions, respectively with the catalytic site of the enzyme, or with its regulatory domain.

10. Kits according to claim 9, **characterized in that** the fluorescent probes comprise a compound capable of interacting with the regulatory domain of PKC are phospholipids constituents of the membrane, proteic or peptidic probes of membranous or cytoskeletal nature, or the derivatives of the substrates of the enzymes PKC or PKA.

## Patentansprüche

1. Verfahren zur Diagnose von Morbus Alzheimer, **dadurch gekennzeichnet, dass** es umfasst:
- die Inkubation einer Blutprobe mit einer fluoreszierenden Sonde, welche die katalytische Stelle des Proteins Kinase C (PKC) und des Proteins Kinase A (PKA) erkennt, oder mit zwei fluoreszierenden Sonden, welche jeweils die katalytische Stelle und die regulierende Domäne eines der beiden Enzyme (PKC und PKA) erkennen, unter Bedingungen, welche die spezifische Verbindung der Sonde oder der Sonden mit PKC oder PKA ermöglichen,
wobei die Sonde, welche die regulierende Domäne des PKC oder des PKA erkennt, Verbindungen aufweist, welche eine Emission im Erregungsbereich der mit der katalytischen Domäne des Enzyms interagierenden Sonde ermöglichen können,
- das Messen der Intensität der Fluoreszenz des erhaltenen Präparats und dessen eventueller Variationen durch Hinzufügen eines Hemmstoffs und/oder eines Aktivators für das PKC oder das PKA, wobei eine Variation der Daten der Fluoreszenzemissionsspektren gegenüber denjenigen der bei gesunden Patienten erhaltenen Spektren eine Unterscheidung in bezug auf Morbus Alzheimer bildet,
wobei das Messen der Intensität der Fluoreszenz des erhaltenen Präparats im Falle einer Inkubation einer Blutprobe mit den beiden fluoreszierenden Sonden, welche jeweils die katalytische Stelle und die regulierende Domäne des PKC oder des PKA erkennen, erfolgt, indem die Probe mit der Absorptionswellenlänge der die katalytische Stelle des Enzyms erkennenden flureszierenden Sonde erregt wird und anschließend das bei der Emissionswellenlänge der katalytischen Sonde erhaltene Signal beobachtet wird, welches eine Unterscheidung in bezug auf Morbus Alzheimer bildet.

2. Verfahren nach Anspruch 1, **dadurch gekennzeichnet, dass** die fluoreszierenden Sonden, welche die katalytische Stelle des PKC oder des PKA erkennen, und diejenigen, welche dessen regulierende Domäne erkennen, eine an ein Fluorophor gekoppelte Verbindung aufweisen, welche in der Lage ist, unter den Inkubationsbedingungen mit der katalytischen Stelle des Enzyms oder mit dessen regulierender Domäne zu interagieren.

3. Verfahren nach Anspruch 2, **dadurch gekennzeichnet, dass** die fluoreszierenden Sonden, welche in der Lage sind, unter den erfindungsgemäßen Bedingungen mit der katalytischen Stelle des PKC zu reagieren, unter organischen Syntheseverbindungen gewählt sind, wie Derivate von Bisindolylmaleimid.

4. Verfahren nach Anspruch 2, **dadurch gekennzeichnet, dass** fluoreszierende Sonden verwendet werden, welche eine Verbindung aufweisen, die in der Lage ist, unter den erfindungsgemäßen Bedingungen mit der regulierenden Domäne des PKC zu interagieren, wie solche Sonden, die aus die Membran bildenden Phospholipiden oder aus Derivaten derartiger Phospholipide gewonnen werden, Eiweiß- oder Peptidsonden von Membran- oder Zytoskelettart, oder Derivate aus Substraten der Enzyme PKC oder PKA.

5. Verfahren nach Anspruch 4, **dadurch gekennzeichnet, dass** die Sonden, welche die regulierende Domäne des PKC oder des PKA erkennen, und die Sonden, welche direkt die katalytische Domäne der Enzyme PKC erkennen, gleichzeitig verwendet werden.

6. Verfahren nach einem der Ansprüche 1 bis 5, **dadurch gekennzeichnet, dass** der Inkubationsschritt bei Umgebungstemperatur durchgeführt wird.

7. Verfahren nach einem der vorhergehenden Ansprüche, **dadurch gekennzeichnet, dass** ein Teil der Probe allein mit der Sonde oder den Sonden zur Inkubation gebracht wird, ein anderer Teil zunächst mit einem PKC- oder PKA-Hemmstoff und anschließend mit der Sonde oder den Sonden zur Inkubation gebracht wird, und ein anderer Teil vorab mit der Sonde oder den Sonden und anschließend mit einem PKC- oder PKA-Aktivator zur Inkubation gebracht wird.

8. Kits oder Testsätze zur Diagnose von Morbus Alzheimer. **Dadurch gekennzeichnet, dass** sie aufweisen:
- eine Gebrauchsanleitung,
- zwei fluoreszierende Sonden, welche jeweils die katalytische Stelle und die regulierende Domäne eines der Enzyme PKC oder PKA erkennen,
und
- Reagenzmittel zur Durchführung des Diagnosetests, wobei die Reagenzmittel unter Aktivatoren und/oder Hemmstoffen für PKC und/oder PKA gewählt sind,
oder, anstelle zweier fluoreszierender Sonden, eine einzelne fluoreszierende Sonde gemäß der vorangehenden Definition mit Hemmstoffen für PKC und/oder PKA als Reagenzmittel.

9. Kits oder Testsätze nach Anspruch 8, **dadurch gekennzeichnet, dass** die Sonden, welche jeweils die katalytische Stelle und die regulierende Domäne eines der genannten Enzyme PKC oder PKA erkennen, eine mit einem Fluorophor gekoppelte Verbindung aufweisen, welche in der Lage ist, unter den Inkubationsbedingungen jeweils mit der katalytischen Stelle des Enzyms oder mit dessen regulierender Domäne zu interagieren.

10. Kits nach Anspruch 9, **dadurch gekennzeichnet, dass** es sich bei den fluoreszierenden Sonden, die eine Verbindung aufweisen, welche in der Lage ist, mit der regulierenden Domäne des PKC zu interagieren, um Phospholipide, die Bestandteil der Membran sind, Eiweiß- oder Peptidsonden von Membran- oder Zytoskelettart, oder um Derivate von Substraten der Enzyme PKC oder PKA handelt.
